# EUROPEAN PATENT SPECIFICATION

(11) **EP 3 585 480 B1**
(45) Date of publication and mention of the grant of the patent: **19.05.2021**
(21) Application number: 18717116.0
(22) Date of filing: 23.02.2018
(51) Int. Cl.: A61N 2/00, A61N 2/06, A61N 5/06, A47C 31/00

(54) **ANTALGIC MAT FOR MAGNETIC THERAPY**
SCHMERZLINDERNDE MATTE FÜR MAGNETISCHE THERAPIE
TAPIS ANTALGIQUE POUR THÉRAPIE MAGNÉTIQUE

(30) Priority: 23.02.2017 IT 201700020814 U
(43) Date of publication of application: 01.01.2020
(73) Proprietor: Sortino, Salvatore, 96019 Rosolini (SR) (IT)
(72) Inventor: Sortino, Salvatore, 96019 Rosolini (SR) (IT)
(74) Representative: Dallaglio, Fabrizio
(86) International application number: PCT/IB2018/051151
(87) International publication number: WO 2018/154511

(56) References cited:
- WO-A1-02/05891
- US-A1- 2004 010 179
- US-A1- 2006 068 660

## Description

### APPLICATION FIELD OF THE INVENTION

The present finding belongs to the field of the treatment of inflammations and physical rebalancing of a person by a series of embodiments, including magnetotherapy.

### STATE OF THE ART

Magnetotherapy is one of the physical therapies with the least number of contraindications, as indicated also by a number of clinical studies carried out with electromagnetic waves.

Magnetotherapy acts at the level of the single cells in the body, "recharging" them with energy.

Living cells, whether nerve or muscle cells, and generally all the cells in a body, have an electric potential, referred to as the electric resting membrane potential (Em), which is calculated by the difference of intra- and extra-cellular electric charge. As a function of the cell type, such value ranges from -55 to -90 mV.

The lower this potential differential is, the lower the cell vitality is. Generally, after a muscle injury, trauma, wound, disease, o other adverse events, the cells surrounding the affected area loose part of their electric charge (i.e., they change their differential potential).

Generally, magnetotherapy requires medium to long treatment times. Typically, it is recommended to carry out cycles of 45-60 days for a minimal duration of 2-3 hours a day. Nonetheless, there are no risks of overdosing, thus it is also possible to carry out a night therapy during several hours without worry.

WO 02/05891 discloses a magnetotherapy system having a bioceramic layer and magnetic bands made of permanent magnets.

US 2004/010179 discloses a magnetotherapy system having a bioceramic layer and emitting pulsing magnetic field.

US 2006/068660 shows a mat with copper and carbon wires. US6520903 refers to a magnetotherapy device with a magnet producing pulsing, monopolar field.

### DISCLOSURE AND ADVANTAGES OF THE FINDING

An object of the present invention is to provide to the art a mat for magnetotherapy treatment in the context of a solution which is simple, reasonable, and quite cost-efficient.

It is noted that the term "mat" used herein should encompass all those devices, such as small mats, blankets, mattress covers, obtained according to the teachings described herein below.

The invention is defined in the claims.

The set of technologies, which are applied to the mat, make the mat a unique object, by virtue of the series of benefits that are achieved by the use thereof. In fact, the mat according to the present invention allows simultaneously acquiring several benefits with only one session, thus in considerably reduced times compared to the known solutions.

Said objects and advantages are all achieved by the mat for magnetotherapy that is the subject-matter of the present finding, which is characterized by what is provided for in the claims set forth below.

### DESCRIPTION OF THE FINDING

The mat which is the subject-matter of the finding is, during use, rested and/or laid on a mattress and possibly secured by means of angular elastics that can be provided for and secured to the mat.

The mat comprises a suitable jack that can be connected in an operative configuration with a magnetotherapy apparatus, i.e., a low or/and high frequency wave generator.

Once the apparatus is activated, it is started. The duration of the applications varies based on the disease and the chronicity thereof (at least 30 minutes a day to a complete 6-hour cycle overnight).

A wire exits the mat, ending in a small metal ball: the energy built up during the das is thus discharged by virtue of metal ball, which is dropped to the ground, where it contacts the floor, in an operative configuration.

By virtue of such solution, the static energy built up during the das is discharged; furthermore, by virtue of the magnetotherapy, a benefit is obtained, resulting from the natural re-polarization of the body during night-time rest.

The mat is obtained with a highly innovative and engineered tissue.

In more detail, the mat is made of a first bioceramic fibre tissue. By way of example, the bioceramic tissue is a tissue to which bioceramic material is added, i.e., biocompatible ceramic materials of various kinds. Magnetic bands for magnetization, specifically, north-south bands. In an embodiment, the magnetic bands are located at the opposite ends of the mat so as to be positioned, during operation, two facing the feet side, and two facing the head side. In other words, the two pairs of bands will be located on longitudinally opposite ends, where longitudinal direction means the direction defined by the head-feet direction of the user.

In addition to the magnetization bands, also central magnetic bands are present. Such magnetic bands are preferably located centrally with respect to the length of the mat, i.e., to the above-mentioned longitudinal direction.

Preferably, the mat comprises at least three central magnetic bands.

According to a preferred embodiment, the central magnetic bands are interposed between the magnetization bands located at the feet side and the head side.

A lower (end) tissue is also present, which is referred to as a "super contact" in the jargon; in other words, it is configured so as to allow connecting the mat to any generator device (low-high frequency) for magnetotherapy.

Engineered yarns are further present, such as, for example, copper fibre, carbon fibre, silver fibre, which are suitably arranged as set forth herein below.

The mat may also comprise a ground discharge system.

The above-mentioned bioceramic fibre tissue is characterized by its mirror function. Each human being emits his/her specific FIR (Far Infra-Red Ray) wave, which is crucial for his/her life processes. In other words, the mirror technology is capable of reflecting for each single individual, his/her own FIR energy waves.

A preferred embodiment provides that the tissue of the mat is composed of at least two different bioceramic fibres. Thanks to this solution, a pain-relieving effect is achieved.

As regards the arrangement of the above-mentioned engineered yarns, it is pointed out that, along at least one edge of the mat, a carbon double wire is present, which is suitable to protect the body against radiations emitted by the electrical equipment with which we are in contact during most of our daily life, contributing to a healthy and pleasant rest

In an embodiment, as regards the arrangement of the above-mentioned engineered yarns, it is pointed out that the upper side of the mat is made of silver fibre, preferably a SILVER LIFE tissue, i.e., a fibre treated with silver ions, (antimicrobial and disinfectant, as well as odour inhibiting properties).

According to another aspect, copper filaments are present in for the complete discharge of the electrostatic energy. The copper or silver wire or tissue is connectable to a polo of a low-high frequency generator. By virtue of such connection, a radio magnetic monopolar field is generated on the tissue, which is usually emitted in a homogeneous manner onto the entire area of interest, or individually or simultaneously with nuclei to generate a low frequency.

The bioceramic technology, with its reflecting ability, recovers the FIR waves emitted by the human body and allows a reintegration by reflecting thereof, and thus to the benefit of arthritic muscular pathologies.

The magnetic technology has the advantage the, through the provision of a north-south polarization, the user does not have to worry about the bed positioning, thus deriving all the possible benefit with the right polar positioning, also hindering the residual radiations and creating a psychophysical balance during sleep .

In an embodiment, the use of magnetic bases with a stable low-intensity field is provided, which concurs to recharge the cells, thus limiting necrosis, hence preventing and creating an antiinflammatory and analgesic action.

In an embodiment, the application of a tissue having a conductive layer and/or wrapped layers for the emission of pulsed electromagnetic waves both at low frequency and high frequency (for a prevention and rebalance) is provided. Such feature allows improving the circulation, resulting in an integral benefit to the user's body, not only at the joint level, but also at the vascular level, moreover regenerating endorphins and melanin to stabilize the brain functions, thus reducing insomnia as well as stress. In addition, one can benefit from the reconditioning and energy generating function, to the benefit of the cellular recharge which thus acts on arthritic, muscular, vascular, and regenerative pathologies.

By the application of the ground discharge system, it is possible to discharge all the static energy built up during the several, challenging everyday activities and, with the suitably applied technology, thus promoting a complete relaxation to the entire body.

In an embodiment, carbon engineered yarn is used on the side parts of the mat, which protects the body against radiations emitted by electrical accessories with which the user can be in contact during most of our everyday life, concurring to a healthy, pleasant rest.

As mentioned above, in the upper part of the mat, the use of a fibre treated with silver ions allows interacting against microbes and bacteria, while providing pest control and disinfection, as well as blocking the absorption of malodours.

The mat can further employ, in addition to what has been stated above, a further tissue composed at least partially of fibres obtained from seaweed extract. The advantage of the seaweed content is the set of vitamins A, B, E, further minerals, such as magnesium, calcium, potassium, and phosphorus, amino acids and microelements that last throughout the tissue life for a benefit to the body and the skin. This has an antioxidant, antiinflammatory, revitalizing and skin protective effect, neutralizes most of the free radicals, referred to as RNS and ROS, which are produced as a function of the circumstances affecting us, such as stresses, improper nutrition, pollution, etc. By virtue of these aspects, further benefits are obtained, such as a slowing down of aging and a reduction of diseases such as atopic dermatites, etc.

## Claims

1. A pain-relieving mat for magnetotherapy of a user, the mat having longitudinally opposite ends referred to as a feet side and a head side, the mat comprising at least:
a. a first tissue composed of at least one bioceramic fibre configured to generate a pain-relieving effect by reflecting FIR waves emitted by the user;
b. two pairs of magnetic bands for a north-south magnetization located at the ends of said mat, said two pairs of magnetic bands comprising two bands being arranged at the feet side and two bands being arranged at the head side;
c. central magnetic bands, located centrally with respect to the longitudinal direction of the mat, preferably being interposed between the magnetic bands at the feed side and the magnetic bands at the head side;
the mat being **characterised by** further comprising:
d. a lower tissue, configured to :
i. connect the mat to a generator configured to generate pulsed high and/or low frequency electromagnetic waves and
ii. generate a monopolar radiofrequency field to be regularly emitted in a homogeneous manner towards the user, and
e. one or more synthetic yarns, selected from a copper fibre, a carbon fibre, and/or a fibre treated with silver ions referred to as a silver fibre.

2. The mat according to claim 1, comprising a wire having at its end a small metal ball that is configured to contact the floor, configured for discharging the energy built up during the day.

3. The mat according to one of the preceding claims, comprising a carbon double wire arranged along at least one edge of the mat.

4. The mat according to one of the preceding claims, wherein the upper side of the mat is made of silver fibre or comprises the silver fibre.

5. The mat according to one of the preceding claims, wherein the first tissue comprises copper filaments for discharging electrostatic energy.

6. The mat according to one of the preceding claims, further comprising a further tissue obtained from the seaweed extract product having an antioxidant, antiinflammatory, revitalizing and skin protective effect, or configured to neutralize free radicals referred to as RNS and ROS.

7. The mat according to one of the preceding claims, wherein the number of the central magnetic bands is three.

8. The mat according to one of the preceding claims, the first tissue being composed of at least two different bioceramic fibres configured to generate a pain-relieving effect.

9. The mat according to one of the preceding claims, wherein the upper side of the mat intended to be in contact with the user comprises said one or more synthetic yarns.

## Patentansprüche

1. Schmerzlinderungsmatte zur Magnetotherapie eines Benutzers, wobei die Matte mit in Längsrichtung gegenüberliegenden Enden versehen ist, die als Fußseite und Kopfseite bezeichnet werden, wobei die Matte mindestens Folgendes umfasst:
a. ein erstes Gewebe, das aus mindestens einer Biokeramikfaser besteht, die konfiguriert ist, um einen schmerzlindernden Effekt mittels einer Reflexion von FIR-Wellen zu erzeugen, die vom Benutzer emittiert werden;
b. zwei Paare von Magnetbändern für die Nord-Süd-Magnetisierung, die sich am Ende der Matte befinden, wobei die zwei Paare von Magnetbändern zwei an der Fußseite angeordnete Bänder und zwei an der Kopfseite angeordnete Bänder umfassen;
c. zentrale Magnetbänder, die zentral in Bezug auf die Längsrichtung der Matte angeordnet sind und vorzugsweise zwischen den Magnetbändern auf der Fußseite und den Magnetbändern auf der Kopfseite angeordnet sind;
wobei die Matte ist **dadurch gekennzeichnet, dass** sie ferner Folgendes umfasst:
d. ein unteres Gewebe, konfiguriert für:
i. Verbinden der Matte mit einem Generator, der zur Erzeugung von gepulsten hoch-und niederfrequenten elektromagnetischen Wellen konfiguriert ist; und
ii. Erzeugen eines monopolaren Hochfrequenzfeldes, das regelmäßig auf homogene Weise an den Benutzer abgegeben wird,
e. ein oder mehrere synthetische Garne, ausgewählt aus einer Kupferfaser, einer Kohlefaser und/oder einer mit Silberionen behandelten Faser, Silberfaser genannt.

2. Matte nach Anspruch 1, umfassend einen Draht, der an seinem Ende mit einer kleinen Metallkugel versehen ist, die zum Kontakt mit dem Boden konfiguriert ist und zum Entladen der während des Tages angesammelten Energie konfiguriert ist.

3. Matte nach einem der vorhergehenden Ansprüche, umfassend einen doppelten Kohlenstoffdraht, der entlang mindestens einer Kante der Matte angeordnet ist.

4. Matte nach einem der vorhergehenden Ansprüche, wobei die Oberseite der Matte aus einer Silberfaser gebildet ist oder die Silberfaser umfasst.

5. Matte nach einem der vorhergehenden Ansprüche, wobei das erste Gewebe Kupferfilamente zum Entladen von elektrostatischen Energie umfasst.

6. Matte nach einem der vorhergehenden Ansprüchen, weiterhin umfassend ein weiteres Gewebe, das aus dem Produkt eines Seetang - Extrakts gewonnen ist und mit einer antioxidativen, entzündungshemmenden, revitalisierenden und hautschützenden Wirkung oder derart konfiguriert ist, um freie Radikale, wie RNS und ROS zu neutralisieren.

7. Matte nach einem der vorhergehenden Ansprüche, wobei die Anzahl der zentralen Magnetbänder gleich drei ist.

8. Matte nach einem der vorhergehenden Ansprüche, wobei das erste Gewebe aus mindestens zwei biokeramischen Fasern besteht, die konfiguriert sind, um eine schmerzlindernde Wirkung zu erzeugen.

9. Matte nach einem der vorhergehenden Ansprüche, wobei die Oberseite der Matte, die mit dem Benutzer in Kontakt kommen soll, ein oder mehrere synthetische Garne umfasst.

## Revendications

1. Tapis antidouleur pour la magnétothérapie d'un utilisateur, le tapis étant pourvu d'extrémités opposées longitudinalement appelées côté pied et côté tête, le tapis comprenant au moins:
a. un premier tissu composé d'au moins une fibre biocéramique configurée pour générer un effet analgésique au moyen d'une réflexion d'ondes FIR émises par l'utilisateur;
b. deux paires de bandes magnétiques à aimantation nord-sud, situées à l'extrémité dudit tapis, lesdites deux paires de bandes magnétiques comprenant deux bandes disposées côté pied et deux bandes disposées côté tête;
c. des bandes magnétiques centrales, situées au centre par rapport à la direction longitudinale du tapis, étant de préférence interposées entre les bandes magnétiques côté pied et les bandes magnétiques côté tête;
le tapis étant **caractérisé en ce qu'**il comprend en outre:
d. un tissu inférieur, configuré pour:
i. connecter le tapis à un générateur configuré pour générer des ondes électromagnétiques pulsée à haute et/ou à basse fréquence et
ii. générer un champ radiofréquence monopolaire à émettre régulièrement de manière homogène vers l'utilisateur, et.
e. un ou plusieurs fils synthétiques, choisis parmi une fibre de cuivre, une fibre de carbone et/ou une fibre traitée aux ions d'argent, appelée fibre d'argent.

2. Tapis selon la revendication 1, comprenant un fil muni à son extrémité d'une petite sphère métallique configurée pour le contact avec le sol, configurée pour décharger l'énergie accumulée au cours de la journée.

3. Tapis selon l'une des revendications précédentes, comprenant un double fil de carbone disposé le long d'au moins un bord du tapis.

4. Tapis selon l'une des revendications précédentes, dans lequel le côté supérieur du tapis est formée d'une fibre d'argent ou comprend la fibre d'argent.

5. Tapis selon l'une des revendications précédentes, dans lequel le premier tissu comprend des filaments de cuivre pour décharger l'énergie électrostatique.

6. Tapis selon l'une des revendications précédentes, comprenant en outre un autre tissu obtenu à partir du produit d'un extrait d'algue à effet antioxydant, anti inflammatoire, revitalisant et protecteur de la peau, ou configuré pour neutraliser les radicaux libres tels que RNS et ROS.

7. Tapis selon l'une des revendications précédentes, dans lequel le nombre de bandes magnétiques centrales est égal à trois.

8. Tapis selon l'une des revendications précédentes, dans lequel le premier tissu est constitué d'au moins deux fibres biocéramiques configurées pour générer un effet analgésique.

9. Tapis selon l'une des revendications précédentes, dans lequel le côté supérieur du tapis destinée à être en contact avec l'utilisateur comprend lesdits un ou plusieurs fils synthétiques.
